(19) Europäisches Patentamt
European Patent Office
Office européen des brevets

(11) EP 4 582 437 A1

(12) **EUROPEAN PATENT APPLICATION**
published in accordance with Art. 153(4) EPC

(43) Date of publication:
**09.07.2025 Bulletin 2025/28**

(21) Application number: **23859496.4**

(22) Date of filing: **01.09.2023**

(51) International Patent Classification (IPC):
***C07K 7/64*** *(2006.01)* ***C07K 7/06*** *(2006.01)*
***C07K 1/13*** *(2006.01)* ***A61K 51/08*** *(2006.01)*
***A61P 35/00*** *(2006.01)*

(52) Cooperative Patent Classification (CPC):
**A61K 51/08; A61P 35/00; C07K 1/10; C07K 1/107; C07K 1/13; C07K 7/06; C07K 7/64**

(86) International application number:
**PCT/CN2023/116515**

(87) International publication number:
**WO 2024/046469 (07.03.2024 Gazette 2024/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **02.09.2022 CN 202211073370**

(71) Applicant: **Hexin (Suzhou) Pharmaceutical Technology Co., Ltd.**
**Suzhou, Jiangsu 215400 (CN)**

(72) Inventors:
- **SHAN, Changyu**
  **Suzhou, Jiangsu 215400 (CN)**
- **ZENG, Dexing**
  **Suzhou, Jiangsu 215400 (CN)**
- **CHEN, Yinfei**
  **Suzhou, Jiangsu 215400 (CN)**
- **LI, Wenjie**
  **Suzhou, Jiangsu 215400 (CN)**

(74) Representative: **Schulz Junghans Patentanwälte PartGmbB**
**Großbeerenstraße 71**
**10963 Berlin (DE)**

Remarks:
The complete document including Reference Table(s) and the Sequence Listing(s) can be downloaded from the EPO website

# (54) CYCLIC PEPTIDE AND PREPARATION METHOD THEREFOR, AND COMPLEX COMPRISING SAME AND USE THEREOF

(57) Provided is a cyclic peptide, having a sequence of cyclo($X^1X^2X^3X^4X^5X^6$), wherein $X^1$ is asparagine; $X^2$ is glycine or sarcosine; $X^3$ is arginine; $X^4$ is selected from a group consisting of threonine, tyrosine, and phenylalanine; $X^5$ is lysine; and $X^6$ is selected from a group consisting of tyrosine, valine, and glutamic acid. Provided is a method for preparing the cyclic peptide. Provided is a complex, comprising the cyclic peptide, a linker, and a chelating agent. Provided is a use of the complex as a radionuclide-labeled targeting molecule. Provided is a radionuclide labeling method, comprising contacting a complex that chelates a radionuclide with an object to be labeled by the radionuclide.

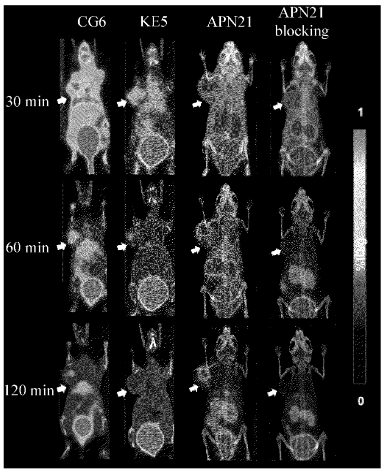


FIG. 3A

EP 4 582 437 A1

## Description

TECHNICAL FIELD

The present disclosure relates to the field of medicine, and more particularly relates to a cyclic peptide and a preparation method therefor, and a complex comprising the same and a use thereof.

BACKGROUND

For quite a long time, therapeutic diagnostics has been a major development direction in the field of nuclear medicine, and radiotherapy diagnostics is also a most mature and widely-applied clinical approach in the field of therapeutic diagnostics. A significant advantage of radiotherapy diagnostics is that a treatment site is also a diagnostic image of lesion of a patient. Imaging is closely associated with therapeutic intervention. Therefore, the development of specific imaging tracers with high affinity and specificity, low non-specific uptake, sufficient retention and effective permeability has become a key research direction in the field of nuclear medicine.

### Aminopeptidase N

Aminopeptidase N (APN, also known as CD13) is a $Zn^{2+}$-dependent membrane-bound metalloproteinase capable of cleaving neutral amino acids from an N-terminus of proteins or polypeptides. CD13 was first purified in 1963 and later shown to be overexpressed in cancer, tumor angiogenesis, and cardiac angiogenesis.

CD13 is expressed in a range of different human cells, such as macrophages, stromal cells, smooth muscle cells, and fibroblasts. Due to its involvement in peptide cleavage, viral infection, endocytosis, and cell signaling, CD13 has been shown to be abnormally overexpressed in various cancers, including breast cancer, ovarian cancer, thyroid cancer, pancreatic cancer, colorectal cancer, and non-small cell lung cancer. In gastric cancer, expression levels of CD13 and TGF-$\beta$1 are associated with tumor size, lymph node metastasis, and tumor differentiation. Similarly, in pancreatic cancer, a serum CD13 level is correlated with tumor size, lymph node metastasis, and metastatic staging. It is a biomarker for early diagnosis and prognosis of pancreatic cancer, and can predict mortality and overall survival of patients with the pancreatic cancer. In colorectal cancer, the higher the CD13 activity in plasma of the patient is, the lower the overall survival rate becomes. In addition, studies have shown that CD13 expression were associated with osteosarcoma, of which immunohistochemistry showed that 77% of the patients with osteosarcoma exhibited CD13 positivity, and high-CD13 expression was linked to poor overall survival in osteosarcoma patients. In summary, CD13 can serve as a biomarker for cancer, useful for clinical detection and evaluation of tumors.

### NGR ligand

Studies have shown that CD13 is not expressed on surfaces of normal blood vessels, but is usually highly expressed in blood vessels undergoing angiogenesis, such as tumor blood vessels, and some peptide structures can bind to an active site of CD13 without being cleaved or degraded by the same. A most well-known peptide sequence is a polypeptide containing asparagine-glycine-arginine (NGR) can target tumor vascular tissues by interacting with CD13. NGR polypeptide was identified in 1998 through phage-displayed peptide library screening technology. Experiments have shown that polypeptides containing NGR structures can bind to CD13 receptor-positive blood vessels in tumor tissues, but cannot bind to other tissues rich in CD13 receptors. The findings further demonstrate the feasibility of using NGR-containing polypeptides as a potential tumor-targeted diagnosis and therapeutic drug.

At present, many studies have taken the polypeptides containing NGR fragment sequences as carriers for delivering chemotherapeutic drugs, nanoparticles, and radioactive isotopes to tumors. Both preclinical and clinical trials have shown that radiolabeled NGR peptides have great potential for tumor vascular diagnostic imaging and targeted radionuclide or ion therapy.

### Cyclic peptide

Cyclic peptide compounds are a class of cyclic compounds with specific structures, broad biological activities and unique mechanisms of action. As a class of peptide molecules with stable and uniform conformation, cyclic peptide compounds have a high selective affinity for receptors and strong metabolic stability. As a drug molecule, cyclic peptide compounds have a wide range of biological activities such as anticancer, antiviral, antibacterial, antifungal, and enzyme inhibition. Therefore, the drug development of cyclic peptides has also attracted more and more attention.

A cyclic structure of cyclic peptides poses conformational restrictions, cyclic peptide compounds generally have a larger surface area, which offers high affinity and recognition specificity with target proteins. The restrictions of

conformational flexibility of a macrocyclic structure also reduce entropy of drug-target binding, thereby improving binding stability. Moreover, a feature of amino acid composition of cyclic peptides determines that cyclic peptide compounds usually have extremely low or even negligible cytotoxicity. In addition, cyclic peptide compounds can be easily prepared through automated chemical synthesis processes, and are amenable to various modifications, treatments, and monitoring, all of these features are greatly conducive to the drug development process.

Currently, the most commonly used cyclic peptide in the study of tumor radiotracers targeting NGR is the cyclo(CNGRC) cyclic peptide. Preliminary studies have used the cyclic peptide to label radionuclides such as $^{99m}Tc$, $^{68}Ga$ and $^{64}Cu$ for molecular imaging of tumor angiogenesis. However, it is worth noting that disulfide bonds in a cyclo(CNGRC) cyclic peptide structure are susceptible to biodegradation or chemical modification, its biological stability and in vivo retention time still need to be further optimized, which limits its use to some extent.

## SUMMARY

In some embodiments, provided is a cyclic peptide, having a sequence of cyclo ($X^1X^2X^3X^4X^5X^6$), wherein,

the $X^1$ is asparagine, particularly L-asparagine or D-asparagine, and particularly L-asparagine;
the $X^2$ is glycine or sarcosine;
the $X^3$ is arginine, particularly L-arginine or L-arginine, and particularly L-arginine;
the $X^4$ is selected from a group consisting of threonine, tyrosine, and phenylalanine, particularly selected from a group consisting of L-threonine, D-threonine, L-tyrosine, D-tyrosine, L-phenylalanine, and D-phenylalanine, particularly threonine, more particularly L-threonine or D-threonine, and more particularly L-threonine;
the $X^5$ is lysine, particularly L-lysine or L-lysine, and particularly L-lysine; and
the $X^6$ is selected from a group consisting of tyrosine, valine, and glutamic acid, particularly selected from a group consisting of L-tyrosine, D-tyrosine, L-valine, D-valine, L-glutamic acid, and D-glutamic acid, particularly tyrosine, more particularly L-tyrosine or D-tyrosine, and more particularly L-tyrosine.

In some embodiments, provided is a method for preparing the cyclic peptide, including: coupling a C-terminus of the $X^5$ to an N-terminus of the $X^6$; coupling a C-terminus of the $X^4$ an N-terminus of the $X^5$; coupling a C-terminus of the $X^3$ an N-terminus of the $X^4$; coupling a C-terminus of the $X^2$ to an N-terminus of the $X^3$; coupling a C-terminus of the $X^1$ to an N-terminus of the $X^2$; and coupling a C-terminus of the $X^6$ to an N-terminus of the $X^1$.

In some embodiments, the method includes performing a condensation reaction of the C-terminus of the $X^5$ with N-terminal protection and the N-terminus of the $X^6$ with the C-terminal protection or coupled to a solid-phase material. In some embodiments, the method includes performing a condensation reaction of the C-terminus of the $X^4$ with N-terminal protection and the N-terminus of the $X^5$ with the C-terminal protection or coupled to a solid-phase material. In some embodiments, the method includes performing a condensation reaction of the C-terminus of the $X^3$ with N-terminal protection and the N-terminus of the $X^4$ with the C-terminal protection or coupled to a solid-phase material. In some embodiments, the method includes performing a condensation reaction of the C-terminus of the $X^2$ with N-terminal protection and the N-terminus of the $X^3$ with the C-terminal protection or coupled to a solid-phase material. In some embodiments, the method includes performing a condensation reaction of the C-terminus of the $X^1$ with N-terminal protection and the N-terminus of the $X^2$ with the C-terminal protection or coupled to a solid-phase material. In some embodiments, the method includes performing a condensation reaction of the C-terminus of the $X^6$ with N-terminal protection and the N-terminus of the $X^1$ with C-terminal protection or coupled to a solid-phase material.

In some embodiments, provided is a complex, comprising the cyclic peptide, a linker, and a chelating agent. In some embodiments, provided is a radionuclide formulation, including the complex and a radionuclide chelated by the chelating agent of the complex.

In some embodiments, provided is a use of the cyclic peptide or the complex of the present disclosure in radionuclide labeling. In some embodiments, provided is a use of the cyclic peptide or the complex of the present disclosure is preparing a radionuclide-labeled targeting molecule. In some embodiments, provided is a use of the cyclic peptide or the complex of the present disclosure in preparing a radionuclide labeling agent. In some embodiments, provided is a use of the cyclic peptide or the complex of the present disclosure in preparing a drug carrier. In some embodiments, provided is a use of the cyclic peptide or the complex of the present disclosure as a drug carrier. In some embodiments, provided is a use of the cyclic peptide, the complex or the radionuclide formulation of the present disclosure in the preparation of a drug for detecting cancer, diagnosing cancer, monitoring cancer progression, monitoring treatment of cancer, or treating cancer. In some embodiments, provided is a use of the cyclic peptide, the complex or the radionuclide formulation of the present disclosure in detecting cancer, diagnosing cancer, monitoring cancer progression, monitoring treatment of cancer, or treating cancer.

In some embodiments, provided is the cyclic peptide or the complex of the present disclosure for radionuclide labeling. In some embodiments, provided is a radionuclide-labeled targeting molecule, including or consisting of the cyclic

peptide or the complex of the present disclosure. In some embodiments, provided is a radionuclide labeling agent, including or consisting of the cyclic peptide or the complex of the present disclosure. In some embodiments, provided is a drug carrier, including or consisting of the cyclic peptide or the complex of the present disclosure. In some embodiments, provided is a formulation for detecting cancer, diagnosing cancer, monitoring cancer progression, monitoring treatment of cancer, or treating cancer, including or consisting of the cyclic peptide, the complex or the radionuclide formulation of the present disclosure. In some embodiments, provided is a drug for detecting cancer, diagnosing cancer, monitoring cancer progression, monitoring treatment of cancer, or treating cancer, including the cyclic peptide, the complex or the radionuclide formulation of the present disclosure.

In some embodiments, provided is a method for radionuclide labeling, including contacting the complex or the radionuclide formulation that chelates the radionuclide with an object to be labeled by the radionuclide. In some embodiments, provided is a method for detecting cancer, diagnosing cancer, monitoring cancer progression, or monitoring treatment of cancer, including: administering the complex or the radionuclide formulation chelated with the radionuclide to a subject for detecting cancer, diagnosing cancer, monitoring cancer progression, or monitoring treatment of cancer; detecting the radionuclide and determining level and location of the radionuclide in the subject; comparing the level and location of the radionuclide with levels and locations of the radionuclide in a same location as other aspects of an unaffected subject or an unaffected part of the subject, wherein, compared with the level and location of the radionuclide in a sample from the radionuclide from the unaffected subject or the unaffected part of the subject, a higher level or different location of the radionuclide in the subject indicates that the subject has cancer, thereby detecting cancer, diagnosing cancer, monitoring cancer progression, or monitoring treatment of cancer.

## BRIEF DESCRIPTION OF THE DRAWINGS

FIG. 1 is an electrospray ionization mass spectrometry of APN21-Bn-SCN-NOTA according to some embodiments of the present disclosure.

FIG. 2 is a radio-HPLC of $^{68}$Ga-APN21-Bn-SCN-NOTA according to some embodiments of the present disclosure.

FIG. 3A shows static PET/CT images of HT1080 tumor-bearing mice injected with only $^{68}$Ga-CG6-Bn-SCN-NOTA (CG6 Group) of the comparative example, injected with only $^{68}$Ga-KE5-Bn-SCN-NOTA (KE5 Group) of the comparative example, injected with only $^{68}$Ga-APN21-Bn-SCN-NOTA (APN21 Group) of the example, and co-injected with $^{68}$Ga-APN21-Bn-SCN-NOTA of the example and unlabeled NGR peptide (APN21-Blocking Group), at 0.5, 1, and 2 hours after injection.

FIG. 3B shows static PET/CT images of HT1080 tumor-bearing mice injected with only $^{68}$Ga-CG6-Bn-SCN-NOTA (CG6 Group) of the comparative example, injected with only $^{68}$Ga-KE5-Bn-SCN-NOTA (KE5 Group) of the comparative example, injected with only $^{68}$Ga-APN21-Bn-SCN-NOTA (APN21 Group) of the example, and co-injected with $^{68}$Ga-APN21-Bn-SCN-NOTA of the example and with unlabeled NGR peptide (APN21-Blocking Group), at 0.5, 1, and 2 hours after injection, and tumor uptake calculated based the $^{68}$Ga-APN21-Bn-SCN-NOTA signal intensity.

## DETAILED DESCRIPTIONS OF THE EMBODIMENTS

As used herein, singular terms refer to one or more than one. For example, "element" or "an element" refers to one element or more than one element.

As used herein, the term "about" refers to approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%. In some cases, the term "about" refers to a numerical variation of plus or minus 20% of the numerical values set forth. For example, "about 50%" refers to within a range of 45%-55%. Numerical ranges recited herein by endpoints include all numbers and fractions subsumed within that range (for example, 1 to 5 includes 1, 1.5, 2, 2.75, 3, 3.90, 4, and 5). It should also be understood that all numbers and fractions thereof are presumed to be modified by the term "about."

The terms "comprising" or "including" are intended to indicate that a combination (such as device, composition, and method) includes the listed elements (such as units of a device, constituents of a composition, or substantial steps of a method), but does not exclude other elements. When defining a composition or method, the phrase "consisting essentially of..." means excluding other elements that are of significant importance to the combination for its intended purpose. Therefore, a combination consisting essentially of the elements defined herein does not exclude other elements that do not substantially affect the fundamental and novel features of the present disclosure. The term "consisting of" means a combination that excludes other elements (constituent components or substantial method steps). Embodiments defined by each of these transitional phrases fall within the scope of the present disclosure.

The term "amino acid" may be used interchangeably with "amino acid residue", and may refer to a free amino acid and/or to an amino acid residue of a peptide. It will be apparent from the context in which the term is used whether it refers to a free amino acid or a residue of a peptide. An "amino acid" as used herein is meant to include both natural and synthetic amino acids, and both D- and L- amino acids. The term "standard amino acid" refers to any of the twenty standard amino acids (including glycine) commonly found in naturally occurring peptides. As used herein, the terms "D-form" and "L-form" amino acids are not intended to exclude achiral amino acids such as glycine, unless otherwise specified. The term "non-standard amino acid residue" refers to any amino acid, other than the standard amino acids, regardless of whether it is prepared synthetically or derived from a natural source. As used herein, "synthetic amino acid" further encompasses chemically modified amino acids, including but not limited to salts, amino acid derivatives (such as amides), and substitutions. Amino acids contained within the peptides of the present disclosure, and particularly at the at the C- or N-terminus, can be modified by methylation, amidation, acetylation or substitution with other chemical groups which can change a peptide's circulating half-life without adversely affecting activity of the peptide. **In** addition, disulfide bonds may be present or absent in the peptides of the present disclosure.

As used herein, the term "pharmaceutical composition" refers to a composition containing at least one active ingredient, where the composition is acceptable for achieving a specific, effective result in mammals (for example, including but not limited to human being). Based on the needs of the skilled artisan, those of ordinary skill in the art will recognize and understand the technology suitable for determining whether an active ingredient exhibits the desired efficacy.

As used herein, the term "pharmaceutically acceptable carrier" refers to a chemical composition to which a suitable compound or derivative can be combined and which, after combination, can be used to administer a suitable compound to a subject.

As used herein, the term "physiologically acceptable" ester or salt refers to an ester or salt form of the active ingredient that is compatible with any other ingredients of a pharmaceutical composition, which is not deleterious to the subject receiving the composition.

As used herein, the term "pharmaceutically acceptable" means physiologically tolerable, for either human or veterinary use.

As used herein, the term "pharmaceutical composition" includes formulations intended for both human and veterinary use.

As used herein, the term "a plurality of" refers to at least two.

As used herein, the term "polynucleotide" refers to a single strand or parallel and antiparallel strands of nucleic acid. Therefore, a polynucleotide may be a single-stranded or double-stranded nucleic acid.

As used herein, the term "polypeptide" refers to a polymer composed of amino acid residues, related naturally occurring structural variants, and synthetic non-naturally occurring analogs thereof linked via peptide bonds, related naturally occurring structural variants, and synthetic non-naturally occurring analogs thereof.

As used herein, the term "synthetic peptides or polypeptides" refers to non-naturally occurring peptides and polypeptides. For example, a synthetic peptide or polypeptide can be synthesized using an automatic peptide synthesizer.

As used herein, the term "N-terminal protection" refers to the protection of an amino group at an N-terminus of the peptide, which is coupled with any of the various N-terminus protecting groups conventionally used in peptide synthesis. As used herein, the term "C-terminal protection" refers to the protection of an amino group at a C-terminus of the peptide, which is coupled with any of the various N-terminus protecting groups conventionally used in peptide synthesis.

As used herein, the term "high expression" refers to an expression level in a subject that is higher than the expression level in a normal individual; specifically, a value or level of a specific substance, such as a specific biomarker or protein, in a biological sample of the subject is higher than the value or level of that substance detected in a biological sample obtained from a healthy or wild-type (normal) individual. As used herein, the term "low expression" refers to an expression level in a subject that is lower than the expression level in a normal individual; specifically, a value or level of a specific substance, such as a specific biomarker or protein, in a biological sample of the subject is lower than the value or level of that substance detected in a biological sample obtained from a healthy or wild-type (normal) individual. Compared with a "normal" expression level or value of a biomarker, the terms "high expression" and "low expression" may indicate "differential levels" or "differential values" or "differential expression," and may include both quantitative and qualitative differences in expression levels.

**In** some embodiments, the $X^1$ is L-asparagine. **In** some embodiments, the $X^1$ is D-asparagine. In some embodiments, the $X^3$ is L-arginine. **In** some embodiments, the $X^3$ is D-arginine. **In** some embodiments, the $X^5$ is L-lysine. **In** some embodiments, the $X^5$ is D-lysine. **In** some embodiments, the $X^4$ is L-threonine. **In** some embodiments, the $X^6$ is L-tyrosine. **In** some embodiments, the $X^4$ is L-tyrosine, and the $X^6$ is L-tyrosine.

In some embodiments, the cyclic peptide is a compound of Formula (I) or derivative thereof. In some embodiments, the cyclic peptide is a compound of Formula (I).

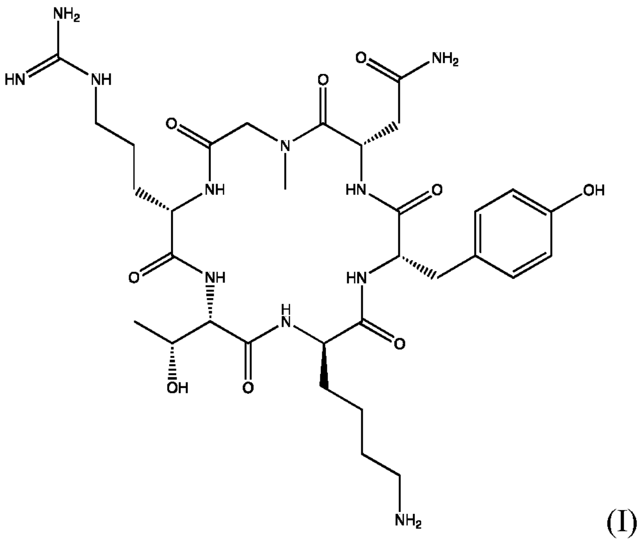

(I)

In some embodiments, the method for preparing the cyclic peptide further includes: deprotecting an N-terminus of the $X^1$ with N-terminal protection to obtain the $X^1$; deprotecting an N-terminus of the $X^2$ with N-terminal protection to obtain the $X^2$; deprotecting an N-terminus of the $X^3$ with N-terminal protection to obtain the $X^3$; deprotecting an N-terminus of the $X^4$ with N-terminal protection to obtain the $X^4$; deprotecting an N-terminus of the $X^5$ with N-terminal protection to obtain the $X^5$; and/or deprotecting an N-terminus of the $X^6$ with N-terminal protection to obtain the $X^6$.

In some embodiments, the method for preparing the cyclic peptide further includes: deprotecting an N-terminus of the $X^1$ with N-terminal protection and C-terminal protection to obtain the $X^1$ with C-terminal protection; deprotecting an N-terminus of the $X^2$ with N-terminal protection and C-terminal protection to obtain the $X^2$ with C-terminal protection; deprotecting an N-terminus of the $X^3$ with N-terminal protection and C-terminal protection to obtain the $X^3$ with C-terminal protection; deprotecting an N-terminus of the $X^4$ with N-terminal protection and C-terminal protection to obtain the $X^4$ with C-terminal protection; deprotecting an N-terminus of the $X^5$ with N-terminal protection and C-terminal protection to obtain the $X^5$ with C-terminal protection; and/or deprotecting an N-terminus of the $X^6$ with N-terminal protection and C-terminal protection to obtain the $X^6$ with C-terminal protection.

In some embodiments, the method for preparing the cyclic peptide further includes: deprotecting a C-terminus of the $X^1$ with N-terminal protection and C-terminal protection to obtain the $X^1$ with N-terminal protection; deprotecting a C-terminus of the $X^2$ with N-terminal protection and C-terminal protection to obtain the $X^2$ with N-terminal protection; deprotecting a C-terminus of the $X^3$ with N-terminal protection and C-terminal protection to obtain the $X^3$ with N-terminal protection; deprotecting a C-terminus of the $X^4$ with N-terminal protection and C-terminal protection to obtain the $X^4$ with N-terminal protection; deprotecting a C-terminus of the $X^5$ with N-terminal protection and C-terminal protection to obtain the $X^5$ with N-terminal protection; and/or deprotecting a C-terminus of the $X^6$ with N-terminal protection and C-terminal protection to obtain the $X^6$ with N-terminal protection.

In some embodiments, the method for preparing the cyclic peptide further includes: deprotecting a C-terminus of the $X^1$ with C-terminal protection to obtain the $X^1$; deprotecting a C-terminus of the $X^2$ with C-terminal protection to obtain the $X^2$; deprotecting a C-terminus of the $X^3$ with C-terminal protection to obtain the $X^3$; deprotecting a C-terminus of the $X^4$ with C-terminal protection to obtain the $X^4$; deprotecting a C-terminus of the $X^5$ with C-terminal protection to obtain the $X^5$; and/or deprotecting a C-terminus of the $X^6$ with C-terminal protection to obtain the $X^6$.

In some embodiments, the method for preparing the cyclic peptide further includes: separating a C-terminus of the $X^1$ coupled to solid-phase material at the C-terminus from the solid-phase material to obtain the $X^1$; separating a C-terminus of the $X^2$ coupled to solid-phase material at the C-terminus from the solid-phase material to obtain the $X^2$; separating a C-terminus of the $X^3$ coupled to solid-phase material at the C-terminus from the solid-phase material to obtain the $X^3$; separating a C-terminus of the $X^4$ coupled to solid-phase material at the C-terminus from the solid-phase material to obtain the $X^4$; separating a C-terminus of the $X^5$ coupled to solid-phase material at the C-terminus from the solid-phase material to obtain the $X^5$ and/or separating a C-terminus of the $X^6$ coupled to solid-phase material at the C-terminus from the solid-phase material to obtain the $X^6$.

In some embodiments, performing a condensation reaction of a C-terminus of the $X^1$ and an N-terminus of the $X^2$, including contacting the $X^1$, $X^2$, HBTU, and DIEA with N-terminal protection. In some embodiments, performing a condensation reaction of a C-terminus of the $X^2$ and an N-terminus of the $X^3$, including contacting the $X^2$, $X^3$, HBTU, and DIEA with N-terminal protection. In some embodiments, performing a condensation reaction of a C-terminus of the $X^3$ and an N-terminus of the $X^4$, including contacting the $X^3$, $X^4$, HBTU, and DIEA with N-terminal protection. In some embodiments, performing a condensation reaction of a C-terminus of the $X^4$ and an N-terminus of the $X^5$, including

contacting the $X^4$, $X^5$, HBTU, and DIEA with N-terminal protection. In some embodiments, performing a condensation reaction of a C-terminus of the $X^5$ and an N-terminus of the $X^6$, including contacting the $X^5$, $X^6$, HBTU, and DIEA with N-terminal protection. In some embodiments, performing a condensation reaction of a C-terminus of the $X^6$ and an N-terminus of the $X^1$, including contacting the $X^6$, $X^1$, HBTU, and DIEA with N-terminal protection.

In some embodiments, the N-terminal protection is Fmoc protection. In some embodiments, the solid-phase material is resin.

In some embodiments, the complex chelates a radionuclide. In some embodiments, the radionuclide includes or consists of at least one selected from a group consisting of $^{44}$Sc, $^{47}$Sc, $^{62}$Cu, $^{64}$Cu, $^{67}$Cu, $^{66}$Ga, $^{67}$Ga, $^{68}$Ga, $^{86}$Y, $^{90}$Y, $^{89}$Zr, $^{99m}$Tc, $^{110m}$In, $^{111}$In, $^{113m}$In, $^{114m}$In, $^{177}$Lu, $^{203}$Pb, $^{212}$Pb, $^{212}$Bi, $^{213}$Bi, and $^{225}$Ac. In some embodiments, the radionuclide includes or consists of at least one selected from a group consisting of $^{44}$Sc, $^{47}$Sc, $^{64}$Cu, $^{67}$Cu, $^{67}$Ga, $^{68}$Ga, $^{90}$Y, $^{99m}$Tc, $^{111}$In, $^{177}$Lu, $^{212}$Pb, $^{213}$Bi, and $^{225}$Ac. In some embodiments, the radionuclide includes or consists of at least one selected from a group consisting of $^{99m}$Tc, $^{68}$Ga, and $^{64}$Cu. In some embodiments, the linker includes or consists of polyethylene glycol (PEG). In some embodiments, the radionuclide is included in some embodiments; and the chelating agent is selected from, and includes or consists of at least one selected from a group consisting of ions formed by reducing one or more hydrogen ions of 1,4,7,10-tetraazacyclododecane-N,N',N",N"'-tetraacetic acid (DOTA), 1,4,7-triazacyclononane-N,N',N"-triacetic acid (NOTA), diethylenetriamine-N,N,N',N",N"-pentaacetic acid (DTPA), 1,4,8,11-tetraazacyclotetradecane-1,4,8,11-tetraacetic acid (TETA), 2,2'-((6-amino-1-(4,7-bis(carboxymethyl)-1,4,7-triazonan-1-yl)hexan-2-yl)azanediyl)diacetic acid (NETA), 1,4,7,10-tetraazacyclododecane-1,4,7-triacetic acid (DO3A), ethylenebis(o-hydroxyphenyl) glycine, (EHPG), N,N'-bis(2-hydroxybenzyl)ethylenediamine-N,N'-diacetic acid (HBED), 1,4,7,10-tetraazacyclododecane-$\alpha,\alpha',\alpha'',\alpha'''$-tetramethyl-N,N',N",N"'-tetraacetic acid (DOTMA), 1,4,8,11-tetraazacyclotetradecane-1,4,8,11-(methyl tetraacetic acid) (TETMA) ethylenediamine tetraacetic acid (FDTA), 1,3-propylenediaminetetraacetic acid (PDTA), triethylene tetraaminehexaacetic acid (TTHA), 1,5,10-N,N',N"-tris(2,3-dihydroxybenzoyl)-tricatecholate (LICAM), 1,3,5-N,N',N"-tris(2,3-dihydroxybenzoyl)aminomethylbenzene (MECAM), and 6-hydrazinonicotinic acid (HYNIC).

In some embodiments, the cyclic peptide is coupled to a linker. In some embodiments, $X^5$ in the cyclic peptide is coupled to a linker. In some embodiments, lysine in the cyclic peptide is coupled to a linker, particularly lysine of $X^5$, particularly an $\varepsilon$-amino group of lysine of $X^5$, is coupled to a linker. In some embodiments, the linker is coupled to the chelating agent. In some embodiments, the linker is coupled to both the cyclic peptide and the chelating agent, particularly, a first end of the linker is coupled to the cyclic peptide, and a second end of the linker, different from the first end, is coupled to the chelating agent.

In some embodiments, the cancer is a tumor with high expression of CD13. In some embodiments, the cancer is selected from a group consisting of head and neck cancer, liver cancer, pancreatic cancer, esophageal cancer, gastric cancer, lung cancer, breast cancer, ovarian cancer, uterine cancer, endometrial cancer, cervical cancer, prostate cancer, adrenal cancer, lymphoma, salivary gland cancer, bone cancer, brain cancer, cerebellar cancer, colon cancer, rectal cancer, colorectal cancer, oronasopharyngeal cancer, kidney cancer, bladder cancer, skin cancer, melanoma, basal cell carcinoma, hard palate cancer, tongue squamous cell cancer, meningioma, pleomorphic adenoma, astrocytoma, soft tissue sarcoma, chondrosarcoma, cortical adenoma, mesothelioma, squamous cell carcinoma, and adenocarcinoma. In some embodiments, the cancer is selected from a group consisting of breast cancer, ovarian cancer, thyroid cancer, pancreatic cancer, colorectal cancer, non-small cell lung cancer, and osteosarcoma. In some embodiments, the cancer is selected from a group consisting of esophageal cancer, pancreatic cancer, and gastric cancer.

A new structure of NGR cyclic peptide is developed to have higher in vivo stability, stronger affinity for a CD13 receptor, stronger targeting ability, and is capable of accurately locating the CD13 receptor in vivo after being labeled with the radionuclide, achieving a purpose of tumor molecular imaging diagnosis via PET imaging.

An objective of targeted tumor therapy is achieved by labeling therapeutic radionuclides.

Provided are a new structure of NGR cyclic peptide, as well as preparation method and use of radiopharmaceutical using the polypeptide. In some embodiments, the radiopharmaceutical and labeling techniques of the present disclosure can be used for molecular imaging and treatment of malignant tumors targeting a CD13 receptor in angiogenesis. In some embodiments, the receptor targeting of the present disclosure is good, the stability is strong, and labeling is simple, thereby having high application value.

In order to further elaborate on the technical means and effects adopted by the present disclosure to achieve the predetermined objects, the specific implementations, structures, features and effects of the present disclosure of the present disclosure are described in detail below in conjunction with the accompanying drawings and preferred embodiments.

## Synthesis of cyclic peptide

Polypeptide was synthesized using a solid-phase fully-automatic continuous flow polypeptide synthesizer from a C-terminus to an N-terminus of a sequence.

1 *n* eq. of a first amino acid (C-terminus amino acid) with 9-fluorenylmethyloxycarbonyl (Fmoc) protecting group

and on CTC resin was weighed and placed in a reactor, and dichloromethane (DCM) was added to swell for half an hour. DCM was removed, a 2% piperidine-N,N'-dimethylformamide (DMF) solution was added to remove the Fmoc protecting group from an N-terminus of the first amino acid on the resin. In some embodiments, in the cyclic peptide with a sequence of cyclo ($X^1X^2X^3X^4X^5X^6$), any of $X^1$, $X^2$, $X^3$, $X^4$, $X^5$, and $X^6$ could be the first amino acid.

3 *n* eq. of a next amino acid, 3 *n* eq. of HBTU (*O*-Benzotriazole-*N*,*N*,*N'*,*N'*-tetramethyluronium hexafluorophosphate), and 10 *n* eq. of DIEA (N,N-diisopropylethylamine) were pumped into the reactor for condensation reaction at reflux for 5 min. The resin was then washed four times with DMF in the reactor. 2% piperidine DMF solution was added to remove the Fmoc protecting group from an N-terminus of the next amino acid, and washed four times with DMF. A polypeptide sequence was completed in this way.

The resin was dried with nitrogen, taken out of a reaction column, and poured into a flask, a certain amount (about 10 mL per gram of resin) of cleavage solution (composed of 30% trifluoroethanol and 70% dichloromethane) was added to the flask, the flask was shaken, and the resin was then filtered out.

A filtrate was obtained, a large amount of ether was then added to the filtrate to precipitate a crude product, the crude product was centrifuged, and washed to obtain a crude product containing a linear polypeptide sequence with a protecting group.

A protected peptide fragment was dissolved in DCM, 2 *n* eq. of PyBop and 10 *n* eq. of DIEA were then added, and a reaction was performed at reflux of 45°C overnight, and the solvent was removed using a rotary evaporator to obtain a cyclic peptide with a protecting group.

A protecting group cleavage solution (composed of 95% trifluoroacetic acid (TFA), 2% dithiothreitol, 2% ethanedithiol, and 1% water) was prepared, added to the vessel of cyclic peptide in the previous step, and shaken for reaction for 120 min.

A filtrate was obtained, a large amount of ether was then added to the filtrate to precipitate a crude product, the crude product was centrifuged, and washed to obtain a crude product with a target cyclic peptide sequence.

The crude product with a target cyclic peptide sequence was purified using high-performance liquid chromatography (HPLC) and lyophilized, and a molecular weight of the target product was confirmed using liquid chromatography-mass spectrometry (LC-MS).

**Evaluation of affinities between the cyclic peptides and CD13 protein**

The binding affinity of the polypeptides in the examples and comparative examples to an HT-1080 cell line was identified through cell uptake studies.

*Culture of human fibrosarcoma cell line HT-1080*

The human fibrosarcoma cell line HT-1080 was cultured in Dulbecco's Modified Eagle Medium (DMEM) (Gibco) mixed with 10% sterile filtered fetal bovine serum (FBS) and 1% penicillin-streptomycin antibiotic solution. The cell culture was maintained in a controlled environment at 37°C and 5% $CO_2$, and subculture was performed every 3-4 days. 24 hours prior to the experiment, cells were seeded in a 96-well plate (5000 cells per well) and cultured overnight until the cells adhered to a wall. Before the experiment, cells were washed twice with 1 mL of phosphate-buffered saline (PBS) to remove a growth medium.

*Preparation of Cy3 fluorescent peptide*

The cyclic peptides in Examples 1-28 (sequences shown in SEQ ID NO: 1-28, respectively, hereinafter referred to as APN1-APN28, collectively referred to as APNs) or the cyclic peptide in Comparative Example 1 (sequence shown in SEQ ID NO: 29, hereinafter referred to as KE5) were subjected to condensation reaction with Cy3-NHS fluorescent dye in a DMF solution. Purification is performed through HPLC. APN-Cy3 fluorescent peptides in Examples 1-28 and KE5-Cy3 fluorescent peptide in Comparative Example 1 were thus prepared.

*Treatment of HT-1080 cells with fluorescentpeptide*

Except for the blank control group, 6 groups treated with KE5-Cy3 fluorescent peptide in Comparative Example 1 were established according to concentration gradients of: 3.125 μmol/L, 6.25 μmol/L, 12.5 μmol/L, 25 μmol/L, 50 μmol/L, and 100 μmol/L, with 6 wells in each group.

HT-1080 cells were treated with KE5-Cy3 fluorescent peptide of Comparative Example 1.

After incubation at 37°C for 30 min, the cells were washed twice with PBS, an intracellular fluorescence intensity was measured using a fluorescence microplate reader (with an emission wavelength of 488 nm, an excitation wavelength of 520 nm) to evaluate the uptake of KE5-Cy3 fluorescent peptide by HT-1080 cells.

10 μL of CCK-8 were added to each group of cells and incubated for 4 h, the cells were then read using a microplate reader (with a wavelength of 490 nm), and errors in fluorescence intensity of each group of cells caused by difference in a number of cells was corrected by CCK-8.

Each group was subjected to the above operation three times to determine an optimal concentration for the uptake of KE5-Cy3 fluorescent peptide by HT-1080 cells.

HT-1080 cells were treated with the APN-Cy3 fluorescent peptide of each of the Examples, wherein a concentration of each APN-Cy3 fluorescent peptide was an optimal concentration determined by KE5-Cy3 fluorescent peptides of Comparative Example 1 according to the uptake by the HT-1080 cells.

After incubation at 37°C for 30 min, the cells were washed twice with PBS, an intracellular fluorescence intensity was measured using a fluorescence microplate reader (with an emission wavelength of 488 nm, an excitation wavelength of 520 nm) to evaluate the uptake of APN-Cy3 fluorescent peptide by HT-1080 cells.

10 μL of CCK-8 were added to each group of cells and incubated for 4 h, the cells were then read using a microplate reader (with a wavelength of 490 nm), and errors in fluorescence intensity of each group of cells caused by difference in a number of cells was corrected by CCK-8.

A relative cell uptake (RCU) for each APN-Cy3 fluorescent peptide relative to the KE5-Cy3 fluorescent peptide was calculated using the following formula:

$$\mathrm{RCU} = \frac{F_{\mathrm{APN-Cy3}}}{F_{\mathrm{KE5-Cy3}}}$$

Referring to Table 1 for specific sequences of the cyclic peptides in Comparative Example 1 and Examples 1-28, as well as the relative cell uptake for APN-Cy3 fluorescent peptide in each of the Examples relative to KE5-Cy3 fluorescent peptide in Comparative Example 1 (that is, a multiples calculated by the cellular uptake of the KE5-Cy3 fluorescent peptide as 1.00).

Table 1 Specific sequences of cyclic peptides and relative cell uptake of fluorescent peptides

| | | name | SEQ ID NO | sequence | molecular weight | RCU |
|---|---|---|---|---|---|---|
| | **Comparative Example 1** | KE5 | SEQ ID NO:29 | cyclo(NGREK) | 557.56 | 1.00 |
| **Group 1** | **Example 1** | APN1 | SEQ ID NO:1 | cyclo(NGRDK) | 570.61 | 0.65 |
| | **Example 2** | APN2 | SEQ ID NO: 2 | cyclo(NGRYK) | 618.70 | 2.58 |
| | **Example 3** | APN3 | SEQ ID NO: 3 | cyclo(NGRTK) | 556.63 | 3.14 |
| | **Example 4** | APN4 | SEQ.IDNO: 4 | cyclo(NGRFK) | 602.70 | 2.91 |
| **Group 2** | **Example 5** | APN5 | SEQ ID NO: 5 | cyclo(NGRTKE) | 685.74 | 3.29 |
| | **Example 6** | APN6 | SEQ ID NO: 6 | cyclo(NGRTKV) | 655.76 | 2.72 |
| | **Example 7** | APN7 | SEQ ID NO: 7 | cyclo(NGRTKF) | 703.80 | 0.56 |
| | **Example 8** | APN8 | SEQ ID NO: 8 | cyclo(NGRTKG) | 613.68 | 1.14 |
| | **Example 9** | APN9 | SEQ ID NO: 9 | cyclo(NGRTKY) | 719.80 | 5.03 |
| **Group 3** | **Example 10** | APN10 | SEQ ID NO: 10 | cyclo(NGRTCE) | 660.70 | 0.77 |
| | **Example 11** | APN11 | SEQ ID NO: 11 | cyclo(NGRTCV) | 630.72 | 0.92 |
| | **Example 12** | APN12 | SEQ ID NO: 12 | cyclo(NGRTCF) | 678.77 | 0.82 |
| | **Example 13** | APN13 | SEQ ID NO: 13 | cyclo(NGRTCG) | 588.64 | 0.49 |
| | **Example 14** | APN14 | SEQ ID NO: 14 | cyclo(NGRTCY) | 694.77 | 1.05 |

(continued)

| | | name | SEQ ID NO | sequence | molecular weight | RCU |
|---|---|---|---|---|---|---|
| Group 4 | **Example 15** | APN15 | SEQ ID NO: 15 | cyclo(NGRTKy) | 719.80 | 4.43 |
| | **Example 16** | APN16 | SEQ ID NO: 16 | cyclo(NGRTKv) | 655.76 | 2.17 |
| | **Example 17** | APN17 | SEQ ID NO: 17 | cyclo(NGRTKt) | 703.80 | 0.49 |
| | **Example 18** | APN18 | SEQ ID NO: 18 | cyclo(NGRtKY) | 719.80 | 2.83 |
| | **Example 19** | APN19 | SEQ ID NO: 19 | cyclo(NGRTKY) | 719.80 | 4.12 |
| Group 5 | **Example 20** | APN20 | SEQ ID NO: 20 | cyclo(*Nme*GRTKY) | 733.83 | 0.78 |
| | **Example 21** | APN21 | SEQ ID NO: 21 | cyclo(N*Sar*RTKY) | 733.83 | 5.68 |
| | **Example 22** | APN22 | SEQ ID NO: 22 | cyclo(N*Sar*RTKy) | 733.83 | 4.98 |
| Group 6 | **Example 23** | APN23 | SEQ ID NO: 23 | cyclo(N*Sar*RT*Orn*Y) | 719.80 | 1.20 |
| | **Example 24** | APN24 | SEQ ID NO: 24 | cyclo(N*Sar*RT*Dab*Y) | 705.77 | 0.57 |
| | **Example 25** | APN25 | SEQ ID NO: 25 | cyclo(N*Sar*RTcY) | 708.79 | 0.72 |
| | **Example 26** | APN26 | SEQ ID NO: 26 | cyclo(*Nme*GRT*Dab*Y) | 705.77 | 0.41 |
| | **Example 27** | APN27 | SEQ ID NO: 27 | cyclo(*Nme*GRT*Orn*Y) | 719.80 | 0.37 |
| | **Example 28** | APN28 | SEQ ID NO: 28 | *cyclo*(*Nme*GRTcY) | 708.79 | 0.62 |

In Table 1, *Orn* represents ornithine; *Dab* represents 2,4-diaminobutyric acid; *Sar* represents sarcosine; *Nme* represents *N*-methyl-asparagine; and lowercase letters represent D-amino acid.

It can be seen that the relative cell uptake of the APN-Cy3 fluorescent peptide in Examples 2, 3, 4, 5, 6, 9, 15, 16, 18, 19, 21, and 22 is at least twice that of the cell uptake of the KE5-Cy3 fluorescent peptide in Comparative Example 1, and the relative cell uptake of the APN-Cy3 fluorescent peptide in Examples 9, 15, 19, 21, and 22 is at least four times that of the cell uptake of the KE5-Cy3 fluorescent peptide. The relative cell uptake of the APN21-Cy3 fluorescent peptide in Example 9 is about 5.03 times that of the KE5-Cy3 fluorescent peptide, and the relative cell uptake of the APN21-Cy3 fluorescent peptide in Example 21, is about 5.68 times that of the KE5-Cy3 fluorescent peptide, indicating that the cyclic peptides can be very effectively taken up by the cells.

As can be seen from Table 1, for the cyclic peptide with the sequence cyclo ($X^1X^2X^3X^4X^5X^6$):

- $X^1$ may be asparagine, of which Cy3 fluorescent peptide can achieve a higher relative cell uptake compared to $X^1$ being N-methyl-asparagine.
- $X^2$ may be glycine or sarcosine, both of which can result in a higher relative cell uptake; specifically, $X^2$ may be particularly sarcosine, of which Cy3 fluorescent peptide thereof can achieve a higher relative cell uptake.
- $X^4$ may be selected from a group consisting of threonine, tyrosine, and phenylalanine, all of which can result in a higher relative cell uptake; specifically, $X^4$ may be threonine, and particularly L-threonine, of which Cy3 fluorescent peptide thereof can achieve a higher relative cell uptake.
- $X^5$ may be lysine, of which Cy3 fluorescent peptide thereof can achieve a higher relative cell uptake compared to $X^5$ being ornithine, 2,4-diaminobutyric acid or cysteine.
- $X^6$ may be selected from a group consisting of tyrosine, valine, and glutamic acid, all of which can result in a higher relative cell uptake; specifically, $X^6$ may be tyrosine, and particularly L-tyrosine, of which Cy3 fluorescent peptide thereof can achieve a higher relative cell uptake.

**Stability study**

The experiment was carried out by using an *in vitro* constant temperature (37°C) incubation method to study the stability of the cyclic peptide APN21 of Example 21 and the cyclic peptide KE5 of Comparative Example 1 in mouse plasma or PBS (1.0 μg/mL). The two cyclic peptides were dissolved in 1 mL of mouse serum, respectively, and incubated at 37°C for 0 h, 0.5 h, 1 h, 4 h, 24 h, and 48 h, and a percentage of drug remaining was then measured and determined. PBS was used as a negative control group. Peak areas of the drug and an internal standard were measured using an LC-MS/MS method, and ratios of the peaks were used instead of the drug concentration for calculation.

Results were shown in Table 2.

Table 2 Percentage of drug remaining of cyclic peptides of Example 21 and Comparative Example 1 after *in vitro* incubation at constant temperature in mouse plasma or PBS

| cyclic peptide | system | percentage of drug remaining | | | | | |
|---|---|---|---|---|---|---|---|
| | | 0 h | 0.5 h | 1 h | 4 h | 24 h | 48 h |
| Comparative Example 1 | mouse plasma | 100 | 94.81 | 89.37 | 80.23 | 39.75 | 12.42 |
| | PBS | 100 | 99.10 | 96.62 | 91.44 | 73.39 | 42.73 |
| Example 21 | mouse plasma | 100 | 99.71 | 98.70 | 95.56 | 88.63 | 87.46 |
| | PBS | 100 | 99.82 | 99.07 | 98.34 | 93.55 | 90.63 |

It can be seen from the table above that compared to the cyclic peptide in Comparative Example 1, the cyclic peptide in Example 21 exhibits a significantly higher percentage of drug remaining after *in vitro* incubation at constant temperature in mouse plasma or PBS, indicating that it has a better stability.

**Animal experiments**

*1. Synthesis of APN21-Bn-SCN-NOTA*

1) 3.8 mg (5.18 μmol) of the cyclic peptide from Example 21 (APN21) was weighed and dissolved in 50 μL of DMF to obtain a DMF solution of APN21. 6.5 mg of N,N-diisopropylethylamine (DIEA) (52 μmol) was added to the solution of APN21.
2) 2.9 mg (5.18 μmol) of NOTA-Bn-SCN ester trihydrochloride was weighed and dissolved in 50 μL of DMF.
3) The NOTA-Bn-SCN solution was added dropwise to the solution of APN21 under oscillation conditions to obtain a reaction mixture, and the reaction mixture was stirred at room temperature for 4 h to obtain a product.
4) The product was separated using high-performance liquid chromatography (HPLC), with a semi-preparative C18 column as a stationary phase and a gradient elution method as a mobile phase at a flow rate of 4 mL/min, changing from 5% acetonitrile to 50% acetonitrile in 18 min.
5) The product was further identified by the HPLC and mass spectrometry. Electrospray ionization mass spectrometry (ESI-MS) result: *m/z* $[M+H]^+$ = 1184.87 (formula: $C_{53}H_{82}N_{12}O_{16}$, calculated molecular weight 1183.54), a mass spectrum was shown in FIG. 1.
6) The target product fraction was lyophilized overnight.

*2. $^{68}$Ga labeling of APN21-PEG$_4$-DOTA*

1) 0.75 μmol of APN21-Bn-SCN-NOTA was dissolved in 1.5 mL of 0.25 M sodium acetate solution.
2) 4 mL of 0.05 M hydrochloric acid solution was used to elute a germanium-gallium generator to prepare an eluent $^{68}GaCl_3$.
3) The eluent of 1 mCi activity was taken and added to the APN21-Bn-SCN-NOTA solution, which was placed in a heater at 60°C to react for 10 min to obtain a $^{68}$Ga-APN21-Bn-SCN-NOTA conjugate. A resulting mixture was monitored and quantitatively labeled by radio-HPLC, with a purity being >97%, and a radio-chromatogram was specifically shown in FIG. 2.

In addition, a $^{68}$Ga-KE5-Bn-SCN-NOTA complex in Comparative Example 1 and a $^{68}$Ga-CG6-Bn-SCN-NOTA complex of Comparative Example 2 were prepared using a method same as the method for preparing the $^{68}$Ga-APN21-Bn-SCN-NOTA complex the above Example 21 (where "CG6" stated herein refers to a cyclic peptide with a sequence of cyclo(CNGRC), as shown in SEQ ID NO: 30).

*3. Modeling of human fibrosarcoma xenograft tumor*

HT-1080 cells were taken from normal NCr nude mice (18-25 g, 4-6 weeks old, n=3), and $2\times10^6$ cells were implanted subcutaneously in right shoulders of mice in a mixture of 200 μL phosphate buffer and matrix gel (v/v, 1/1). After an average of 1.5 weeks, a tumor diameter reached about 10 mm, which was sufficient for biodistribution and PET imaging

studies.

*4. Positron emission tomography/computed tomography (PET/CT) imaging of small animals*

PET/CT and image analysis were performed using a small-animal NovclMedcal PET/CT scanner (Novel Medical Equipment Ltd., Beijing). Maximum tangential and radial half-widths of the scanner in a center of field of view were 1.5 mm, and at an edge of the field of view were 1.8 mm.

For CG6 Group, about 3.7 MBq (100 $\mu$Ci) of the $^{68}$Ga-CG6-Bn-SCN-NOTA conjugate of Comparative Example 2 was injected into HT-1080 tumor-bearing mice via tail vein under isoflurane anesthesia. Static PET/CT images were obtained for 15 min at 0.5, 1, and 2 hours after intravenous injection.

For KE5 Group, about 3.7 MBq (100 $\mu$Ci) of the $^{68}$Ga-KE5-Bn-SCN-NOTA conjugate of Comparative Example 1 was injected into HT-1080 tumor-bearing mice via tail vein under isoflurane anesthesia. Static PET/CT images were obtained for 15 min at 0.5, 1, and 2 hours after intravenous injection.

For APN21 Group, about 3.7 MBq (100 $\mu$Ci) of the $^{68}$Ga-APN21-Bn-SCN-NOTA conjugate of Example 21 was injected into HT-1080 tumor-bearing mice via tail vein under isoflurane anesthesia. Static PET/CT images were obtained for 15 min at 0.5, 1, and 2 hours after intravenous injection.

For APN21-Blocking Group, about 3.7 MBq (100 $\mu$Ci) of the $^{68}$Ga-APN21-Bn-SCN-NOTA conjugate of Example 21 and unlabeled NGR peptide (15 mg/kg each peptide) were co-injected into HT-1080 tumor-bearing mice (n=3 for each group) under isoflurane anesthesia. Static PET/CT images were obtained for 15 min at 0.5, 1, and 2 hours after intravenous injection.

Please refer to FIG. 3A and 3B, which were the static PET/CT images of HT1080 tumor-bearing mice injected with only $^{68}$Ga-CG6-Bn-SCN-NOTA (CG6 Group), injected with only $^{68}$Ga-KE5-Bn-SCN-NOTA (KE5 group), injected with only $^{68}$Ga-APN21-Bn-SCN-NOTA (APN21 Group), and co-injected with the $^{68}$Ga-APN21-Bn-SCN-NOTA and unlabeled NGR peptide (APN21-Blocking Group) at 0.5, 1, and 2 hours after injection, and tumor uptake calculated based on the $^{68}$Ga-APN21-Bn-SCN-NOTA signal intensity. As shown in FIGs. 3A and 3B, the signal intensity of the $^{68}$Ga-APN21-Bn-SCN-NOTA in APN21-Blocking Group was significantly weaker than that of the unblocked APN21 group and was closer to that of KE5 group. Therefore, it can be demonstrated that target sides of the $^{68}$Ga-APN21-Bn-SCN-NOTA were blocked when the NGR peptide blocked the CD13 receptors, demonstrating the targeting of the $^{68}$Ga-APN21-Bn-SCN-NOTA to the CD13 receptors.

PET and CT images were acquired using NMSoft workstation software (Novel Medical Equipment Ltd., Beijing), and data were given as a percentage of injected dose per gram of tissue or organ (ID/g), and were determined through decay correction for each sample (standardized to a known weight of the injected dose).

The above embodiments are merely preferred embodiments of the present disclosure and cannot be used to limit the scope of protection of the present disclosure, and any non-substantial variations and substitutions made by those skilled in the art on the basis of the present disclosure shall fall within the scope of protection claimed by the present disclosure.

## Claims

**1.** A cyclic peptide, having a sequence of cyclo ($X^1X^2X^3X^4X^5X^6$), wherein

the $X^1$ is asparagine;
the $X^2$ is glycine or sarcosine;
the $X^3$ is arginine;
the $X^4$ is selected from a group consisting of threonine, tyrosine, and phenylalanine;
the $X^5$ is lysine; and
the $X^6$ is selected from a group consisting of tyrosine, valine, and glutamic acid.

**2.** The cyclic peptide according to claim 1, wherein

the $X^4$ is L-threonine or D-threonine, and/or
the $X^6$ is L-tyrosine or D-tyrosine.

**3.** The cyclic peptide according to claims 1 or 2, wherein

the $X^1$ is L-asparagine;
the $X^3$ is L-arginine;

the $X^4$ is L-threonine;
the $X^5$ is L-lysine; and/or
the $X^6$ is L-tyrosine;
optionally, the $X^1$ is L-asparagine, the $X^2$ is sarcosine, the $X^3$ is L-arginine, the $X^4$ is L-threonine, the $X^5$ is L-lysine, and the $X^6$ is L-tyrosine;
optionally, the $X^1$ is L-asparagine, the $X^2$ is glycine, the $X^3$ is L-arginine, the $X^4$ is L-threonine, the $X^5$ is L-lysine, and the $X^6$ is L-tyrosine; or
optionally, the $X^1$ is L-asparagine, the $X^2$ is sarcosine, the $X^3$ is L-arginine, the $X^4$ is L-threonine, the $X^5$ is L-lysine, and the $X^6$ is L-tyrosine.

**4.** A complex, comprising:

the cyclic peptide according to any one of claims 1-3;
a linker, coupled to the cyclic peptide; and
a chelating agent, coupled to the linker.

**5.** The complex according to claim 4, wherein

the linker is polyethylene glycol; and/or
the chelating agent is selected from, and comprises or consists of at least one selected from a group consisting of ions formed by reducing one or more hydrogen ions of 1,4,7,10-tetraazacyclododecane-N,N',N",N'"-tetraacetic acid, 1,4,7-triazacyclononane-N,N',N"-triacetic acid, diethylenetriamine-N,N,N',N",N"-pentaacetic acid, 1,4,8,11-tetraazacyclotetradecane-1,4,8,11-tetraacetic acid, 2,2'-((6-amino-1-(4,7-bis(carboxymethyl)-1,4,7-triazonan-1-yl)hexan-2-yl)azanediyl)diacetic acid, 1,4,7,10-tetraazacyclododecane-1,4,7-triacetic acid, ethylenebis(o-hydroxyphenyl)glycine, N,N'-bis(2-hydroxybenzyl)ethylenediamine-N,N'-diacetic acid, 1,4,7,10-tetraazacyclododecane-α,α',α",α'"-tetramethyl-N,N',N",N'"-tetraacetic acid, 1,4,8,11-tetraazacyclotetradecane-1,4,8,11-(methyltetraacetic acid) ethylenediaminetetraacetic acid (FDTA), 1,3-propylenediaminetetraacetic acid (PDTA), triethylenetetraaminehexaacetic acid (TTHA), 1,5,10-N,N',N"-tris(2,3-dihydroxybenzoyl)-tricatecholate, 1,3,5-N,N',N"-tris(2,3-dihydroxybenzoyl)aminomethylbenzene, and 6-hydrazinonicotinic acid;
optionally, the chelating agent comprises 1,4,7,10-tetraazacyclododecane-N,N',N",N"'-tetraacetic acid.

**6.** Use of the cyclic peptide according to any one of claims 1-3, or the complex according to any one of claims 4-5 in radionuclide labeling, preparing a radionuclide labeling agent or preparing a drug carrier.

**7.** A radionuclide formulation, comprising:

the cyclic peptide according to any one of claims 4-5; and
a radionuclide chelated by the chelating agent of the complex.

**8.** The radionuclide formulation according to claim 7, wherein the radionuclide is selected from at least one of a group consisting of $^{44}Sc$, $^{47}Sc$, $^{64}Cu$, $^{67}Cu$, $^{67}Ga$, $^{68}Ga$, $^{99m}Tc$, $^{90}Y$, $^{111}In$, $^{177}Lu$, $^{212}Pb$, $^{213}Bi$, and $^{225}Ac$.

**9.** Use of the cyclic peptide according to any one of claims 1-4, or the complex according to any one of claims 5-6, or the radionuclide formulation according to any one of claims 7-8 in the preparation of a drug for detecting cancer, diagnosing cancer, monitoring cancer progression, monitoring treatment of cancer, or treating cancer;
optionally, the cancer is selected from a group consisting of head and neck cancer, liver cancer, pancreatic cancer, esophageal cancer, gastric cancer, lung cancer, breast cancer, ovarian cancer, uterine cancer, endometrial cancer, cervical cancer, prostate cancer, adrenal cancer, lymphoma, salivary gland cancer, bone cancer, brain cancer, cerebellar cancer, colon cancer, rectal cancer, colorectal cancer, oronasopharyngeal cancer, kidney cancer, bladder cancer, skin cancer, melanoma, basal cell carcinoma, hard palate cancer, tongue squamous cell cancer, meningioma, pleomorphic adenoma, astrocytoma, soft tissue sarcoma, chondrosarcoma, cortical adenoma, mesothelioma, squamous cell carcinoma, and adenocarcinoma.

**10.** The use according to claim 9, wherein

the cancer is a tumor with high expression of CD13; and/or
the cancer is selected from a group consisting of breast cancer, ovarian cancer, thyroid cancer, pancreatic cancer, colorectal cancer, non-small cell lung cancer, and osteosarcoma.

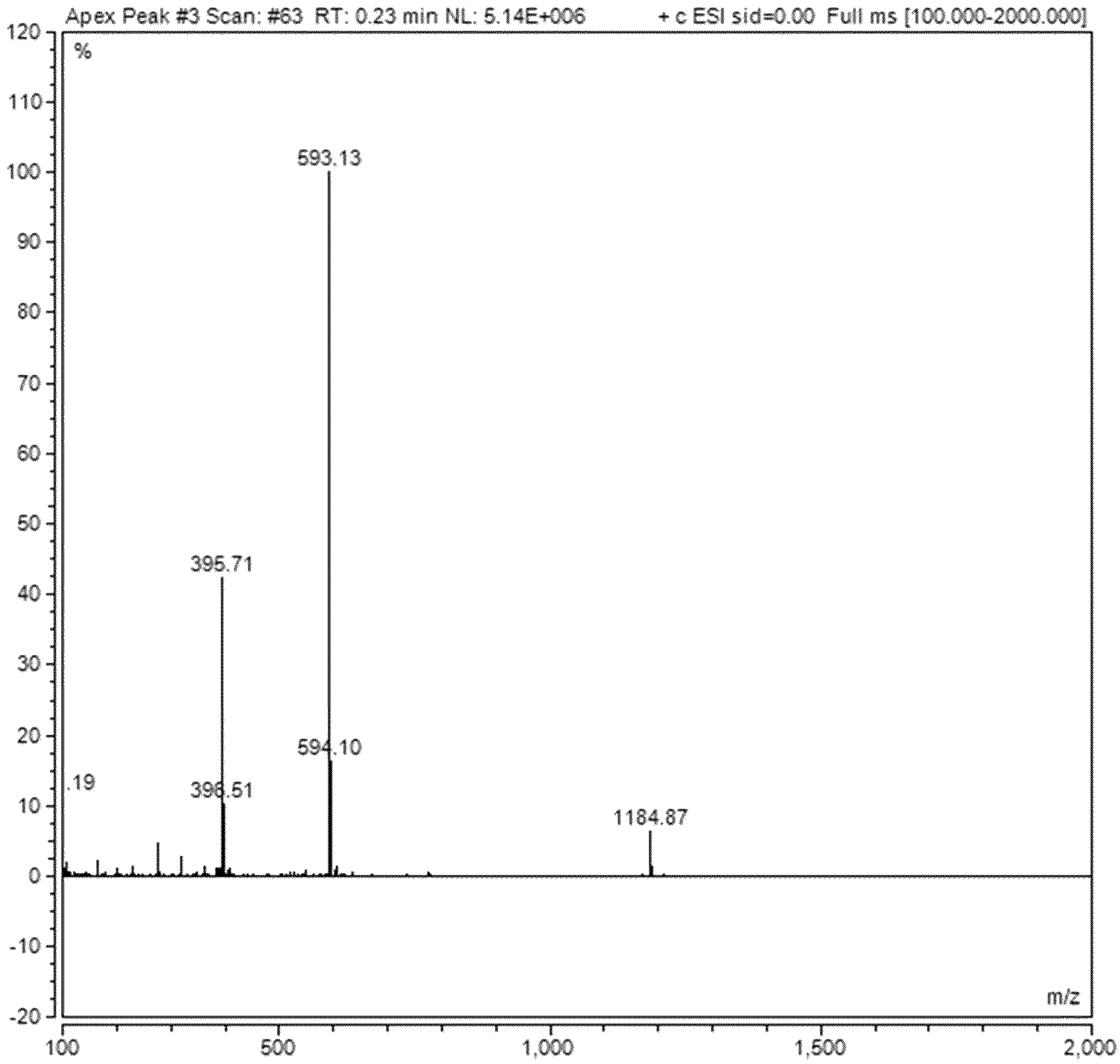
Apex Peak #3 Scan: #63 RT: 0.23 min NL: 5.14E+006
+ c ESI sid=0.00 Full ms [100.000-2000.000]
%
593.13
395.71
594.10
396.51
.19
1184.87
m/z
120
110
100
90
80
70
60
50
40
30
20
10
0
-10
-20
100
500
1,000
1,500
2,000

FIG. 1

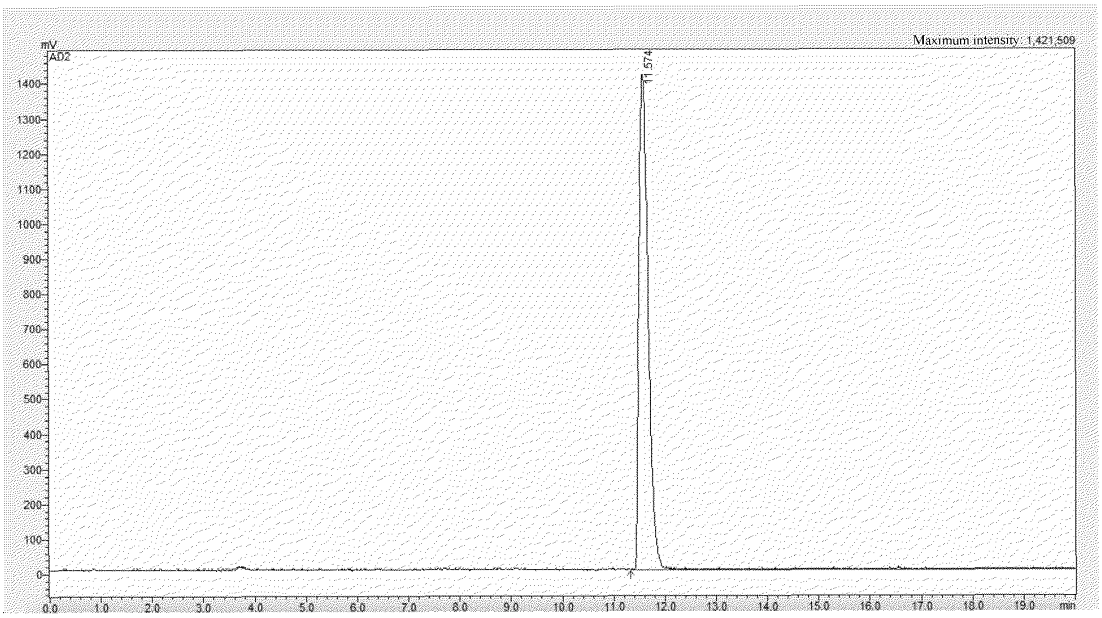
mV
AD2
Maximum intensity: 1,421,509
11.574
1400
1300
1200
1100
1000
900
800
700
600
500
400
300
200
100
0
0.0
1.0
2.0
3.0
4.0
5.0
6.0
7.0
8.0
9.0
10.0
11.0
12.0
13.0
14.0
15.0
16.0
17.0
18.0
19.0
min

FIG. 2

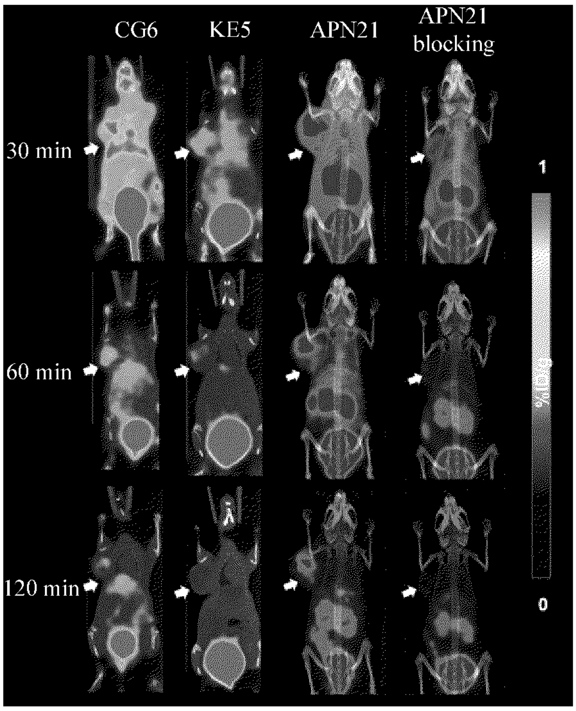
CG6
KE5
APN21
APN21 blocking
30 min
60 min
120 min
1
0
%ID/g

FIG. 3A

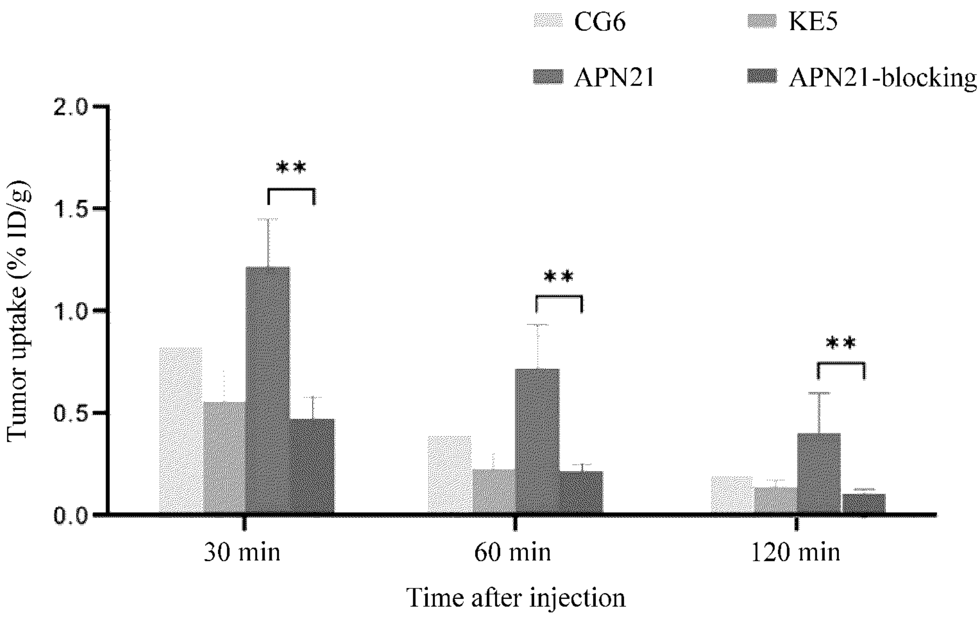
CG6
KE5
APN21
APN21-blocking
Tumor uptake (% ID/g)
2.0
1.5
1.0
0.5
0.0
**
**
**
30 min
60 min
120 min
Time after injection

FIG. 3B

**INTERNATIONAL SEARCH REPORT**

International application No.
**PCT/CN2023/116515**

**A. CLASSIFICATION OF SUBJECT MATTER**

C07K7/64(2006.01)i; C07K7/06(2006.01)i; C07K1/13(2006.01)i; A61K51/08(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07K, A61K, A61P

Documentation searched other than minimum documentation to the extent that such documents are included in the fields searched

Electronic data base consulted during the international search (name of data base and, where practicable, search terms used)

CNABS, CNTXT, ENTXT, VEN, WPABS, DWPI, CNKI, 万方, WANFANG, EBI, NCBI, 百度, BAIDU, ISI Web of Knowledge, PUBMED, STN, 中国专利生物序列检索系统, China Patent Biological Sequence Search System: 核欣(苏州)医药科技有限公司, 单长宇, 曾德兴, 陈银飞, 李文杰, 氨肽酶N, APN, CD13, 环肽, NGR, cyclo(CNGRC), cyclo(NGRTKY), NGRTKY, NSarRTKY, cyclo(NSarRTKY), 天冬酰胺, 甘氨酸, 肌氨酸, 精氨酸, 苏氨酸, 赖氨酸, 酪氨酸

**C. DOCUMENTS CONSIDERED TO BE RELEVANT**

| Category* | Citation of document, with indication, where appropriate, of the relevant passages | Relevant to claim No. |
|---|---|---|
| A | CN 112079900 A (INSTITUTE OF NUCLEAR PHYSICS & CHEMISTRY, CHINA ACADEMY OF ENGINEERING PHYSICS) 15 December 2020 (2020-12-15)<br>entire document | 1-10 (in part) |
| A | CN 103483422 A (FOURTH MILITARY MEDICAL UNIVERSITY OF CHINESE PEOPLE'S LIBERATION ARMY) 01 January 2014 (2014-01-01)<br>entire document | 1-10 (in part) |
| A | CN 103948947 A (NANJING FIRST HOSPITAL et al.) 30 July 2014 (2014-07-30)<br>entire document | 1-10 (in part) |
| A | CN 104984371 A (SHANGHAI JIAO TONG UNIVERSITY) 21 October 2015 (2015-10-21)<br>entire document | 1-10 (in part) |
| A | CN 114773433 A (BEIJING CANCER HOSPITAL (PEKING UNIVERSITY CANCER HOSPITAL)) 22 July 2022 (2022-07-22)<br>entire document | 1-10 (in part) |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 November 2023** | **21 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2023/116515**

**C. DOCUMENTS CONSIDERED TO BE RELEVANT**

| Category* | Citation of document, with indication, where appropriate, of the relevant passages | Relevant to claim No. |
|---|---|---|
| A | US 2015045302 A1 (OSPEDALE SAN RAFFAELE SRL) 12 February 2015 (2015-02-12)<br>entire document | 1-10 (in part) |
| A | WO 2012045719 A2 (MOLMED SPA) 12 April 2012 (2012-04-12)<br>entire document | 1-10 (in part) |

Form PCT/ISA/210 (second sheet) (July 2022)

**INTERNATIONAL SEARCH REPORT**

International application No.
**PCT/CN2023/116515**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/116515**

**Box No. III Observations where unity of invention is lacking (Continuation of item 3 of first sheet)**

This International Searching Authority found multiple inventions in this international application, as follows:

Invention 1: the technical solutions of claims 1-10 that relate to the $X^2$ being glycine, the $X^4$ being threonine, and $X^6$ being tyrosine.

Invention 2: the technical solutions of claims 1-10 that relate to the $X^2$ being sarcosine, the $X^4$ being threonine, and $X^6$ being tyrosine.

Invention 3: the technical solutions of claims 1-10 that relate to the $X^2$ being glycine, the $X^4$ being tyrosine, and $X^6$ being tyrosine.

Invention 4: the technical solutions of claims 1-10 that relate to the $X^2$ being glycine, the $X^4$ being phenylalanine, and $X^6$ being tyrosine.

…

Invention 18: the technical solutions of claims 1-10 that relate to the $X^2$ being sarcosine, the $X^4$ being phenylalanine, and $X^6$ being glutamic acid.

The same technical features of inventions 1-18 are: $X^1$ being asparagine, $X^3$ being arginine, and $X^5$ being a cyclic hexapeptide of lysine. First, the preparation of the cyclic hexapeptide is a conventional technique in the art. There is no evidence in the present application to prove that, in the cyclic hexapeptide, $X^1$ being asparagine, $X^3$ being arginine, and $X^5$ being lysine are necessary and sufficient technical features for exerting the technical effect thereof; that is, it can be seen from the application document that not all the features of $X^1$ being asparagine, $X^3$ being arginine, and $X^5$ being a hexapeptide of lysine can solve the technical problem of the present application. Therefore, inventions 1-18 do not have a same or corresponding special technical feature, and thus do not comply with PCT Rule 13(2).

1. ☐ As all required additional search fees were timely paid by the applicant, this international search report covers all searchable claims.

2. ☐ As all searchable claims could be searched without effort justifying additional fees, this Authority did not invite payment of additional fees.

3. ☑ As only some of the required additional search fees were timely paid by the applicant, this international search report covers only those claims for which fees were paid, specifically claims Nos.: **Invention 1: the technical solutions of claims 1-10 that relate to the X2 being glycine, the X4 being threonine, and X6 being tyrosine; and Invention 2: the technical solutions of claims 1-10 that relate to the X2 being sarcosine, the X4 being threonine, and X6 being tyrosine.**

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.
**PCT/CN2023/116515**

| Patent document cited in search report | | | Publication date (day/month/year) | Patent family member(s) | | | Publication date (day/month/year) |
|---|---|---|---|---|---|---|---|
| CN | 112079900 | A | 15 December 2020 | None | | | |
| CN | 103483422 | A | 01 January 2014 | None | | | |
| CN | 103948947 | A | 30 July 2014 | None | | | |
| CN | 104984371 | A | 21 October 2015 | None | | | |
| CN | 114773433 | A | 22 July 2022 | None | | | |
| US | 2015045302 | A1 | 12 February 2015 | EP | 2827908 | A2 | 28 January 2015 |
| | | | | US | 9561289 | B2 | 07 February 2017 |
| | | | | WO | 2013140317 | A2 | 26 September 2013 |
| | | | | WO | 2013140317 | A3 | 23 January 2014 |
| | | | | GB | 201204868 | D0 | 02 May 2012 |
| WO | 2012045719 | A2 | 12 April 2012 | WO | 2012045719 | A3 | 21 June 2012 |

Form PCT/ISA/210 (patent family annex) (July 2022)